# EUROPEAN PATENT APPLICATION

(11) **EP 4 796 126 A1**
(43) Date of publication of application: **26.08.2026**
(21) Application number: 24878999.2
(22) Date of filing: 15.10.2024
(51) Int. Cl.: A61K 45/06, C07K 16/30, A61P 35/00, A61K 39/395

(54) **COMBINATION OF CLDN18_2 ANTIBODY AND CHEMOTHERAPEUTIC DRUG AND USE THEREOF**

(30) Priority: 16.10.2023 CN 202311331706
(71) Applicant: Jiangsu Aosaikang Biopharmaceutical Co., Ltd., Nanjing, Jiangsu 211112 (CN)
(72) Inventor: SUN, Jiye, Nanjing, Jiangsu 211112 (CN)
(74) Representative: Cohausz & Florack
(86) International application number: PCT/CN2024/124975
(87) International publication number: WO 2025/082356

(57) **Abstract**

The use of a combination of a Claudin 18.2 antibody and a chemotherapeutic drug for the preparation of a medicament for treating a tumor disease. The combination optionally further comprises an immune checkpoint inhibitor.

## Description

### TECHNICAL FIELD

The present invention relates to the use of a combination of a Claudin 18.2 antibody and a chemotherapeutic drug in the preparation of a medicament for the treatment of a tumor disease.

### BACKGROUND

Gastric cancer is one of the major malignant tumors that threaten human life and health. Data shows that in 2020, there were 479,000 new cases and 374,000 deaths of gastric cancer in China. From 2003 to 2015, the 5-year relative survival rate of gastric cancer in China slightly increased from 27.4% to 35.1%. (China Guideline for the Screening, Early Detection and Early Treatment of Gastric Cancer (2022), National Cancer Center of China, http://chinancpcn.org.cn). These statistics clearly demonstrate that the limited treatment regimens for gastric cancer are far from meeting the medical needs.

Antibodies are a relatively new class of targeted therapeutic medicaments that have been widely used in the treatment of various cancers. Antibody-targeted therapies show the potential for higher specificity and reduced side effects as compared to many traditional non-antibody types of cancer therapies. Claudin 18.2 has recently been identified as a target for antibodies for the treatment of gastric cancer and esophageal cancer (J Hematol Oncol. 2017 (1):105). Also, it is a target for the development of antibody medicaments against pancreatic cancer. At present, there are many anti-Claudin 18.2 monoclonal antibodies, such as Zolbetuximab (IMAB362), ASKB589 and Osemitamab (TST001), under clinical development.

Cell tight junction proteins (Claudins) are important molecules that constitute cell tight junctions, which determine the permeability of epithelial cells and also function to block the diffusion of proteins and lipids on the cell membrane surface. Claudin 18 belongs to the Claudin family of proteins and is encoded by the Claudin 18 gene in a human body. The human Claudin 18 gene has two alternative first exons, and through alternative splicing after transcription, generates two protein isoforms, Claudin 18.1 and Claudin 18.2, each with a different sequence only at the N-terminal. Claudin 18.1 and Claudin 18.2 exhibit tissue-specific expression in humans. Claudin 18.1 is predominantly expressed in lung tissues and is not expressed in gastric tissues or gastric cancer. Claudin 18.2, as a highly specific cell surface molecule, is expressed exclusively on differentiated epithelial cells of the gastric mucosa in normal tissues. Claudin 18.2 molecules are expressed in most primary gastric cancers and their metastatic cancers. Due to its high expression specificity in normal tissues and activated expression in a variety of cancers, Claudin 18.2 has become a highly promising target for epithelial tumors.

### BRIEF SUMMARY

In a first aspect, the present invention provides a use of a combination of a Claudin 18.2 antibody and a chemotherapeutic drug in the preparation of a medicament for the treatment of a tumor disease,
the Claudin 18.2 antibody has a heavy chain CDR1 as set forth in SEQ ID NO: 1, a heavy chain CDR2 as set forth in SEQ ID NO: 2, a heavy chain CDR3 as set forth in SEQ ID NO: 3, a light chain CDR1 as set forth in SEQ ID NO: 4, a light chain CDR2 as set forth in SEQ ID NO: 5, and a light chain CDR3 as set forth in SEQ ID NO: 6;
the chemotherapeutic drug is one or more selected from the group consisting of a pyrimidine analogue-based chemotherapeutic drug, a platinum-based chemotherapeutic drug, a paclitaxel-based chemotherapeutic drug, and a camptothecin-based chemotherapeutic drug; and
the combination optionally further includes an immune checkpoint inhibitor.

In a second aspect, the present invention provides a method for the treatment of a tumor disease in a patient, including administering a combination of a Claudin 18.2 antibody and a chemotherapeutic drug to the patient, characterized in that
the Claudin 18.2 antibody has a heavy chain CDR1 as set forth in SEQ ID NO: 1, a heavy chain CDR2 as set forth in SEQ ID NO: 2, a heavy chain CDR3 as set forth in SEQ ID NO: 3, a light chain CDR1 as set forth in SEQ ID NO: 4, a light chain CDR2 as set forth in SEQ ID NO: 5, and a light chain CDR3 as set forth in SEQ ID NO: 6;
the chemotherapeutic drug is one or more selected from the group consisting of a pyrimidine analogue-based chemotherapeutic drug, a platinum-based chemotherapeutic drug, a paclitaxel-based chemotherapeutic drug, and a camptothecin-based chemotherapeutic drug; and
the combination optionally further includes an immune checkpoint inhibitor.

In a third aspect, the present invention provides a pharmaceutical combination for use in the treatment of a tumor disease, including a Claudin 18.2 antibody and a chemotherapeutic drug, characterized in that
the Claudin 18.2 antibody has a heavy chain CDR1 as set forth in SEQ ID NO: 1, a heavy chain CDR2 as set forth in SEQ ID NO: 2, a heavy chain CDR3 as set forth in SEQ ID NO: 3, a light chain CDR1 as set forth in SEQ ID NO: 4, a light chain CDR2 as set forth in SEQ ID NO: 5, and a light chain CDR3 as set forth in SEQ ID NO: 6;
the chemotherapeutic drug is one or more selected from the group consisting of a pyrimidine analogue-based chemotherapeutic drug, a platinum-based chemotherapeutic drug, a paclitaxel-based chemotherapeutic drug, and a camptothecin-based chemotherapeutic drug; and
the pharmaceutical combination optionally further includes an immune checkpoint inhibitor.

In one embodiment of the present invention, the Claudin 18.2 antibody has a heavy chain CDR1 as set forth in SEQ ID NO: 1, a heavy chain CDR2 as set forth in SEQ ID NO: 2, a heavy chain CDR3 as set forth in SEQ ID NO: 3, a light chain CDR1 as set forth in SEQ ID NO: 4, a light chain CDR2 as set forth in SEQ ID NO: 5, and a light chain CDR3 as set forth in SEQ ID NO: 6. In one embodiment of the present invention, the Claudin 18.2 antibody has a heavy chain variable region as set forth in SEQ ID NO: 7, and a light chain variable region as set forth in SEQ ID NO: 8. In one embodiment of the present invention, the Claudin 18.2 antibody has a heavy chain as set forth in SEQ ID NO: 9, and a light chain as set forth in SEQ ID NO: 10.

In one embodiment of the present invention, the chemotherapeutic drug includes pyrimidine analogue-based chemotherapeutic drugs and platinum-based chemotherapeutic drugs. Chemotherapy, referred to as chemo for short, is a therapeutic method that uses chemotherapeutic drugs to kill tumor cells and inhibit the growth of tumor cells.

In one embodiment of the present invention, the pyrimidine analogue-based chemotherapeutic drug is selected from the group consisting of cytarabine, 5-fluorouracil (also called fluorouracil, or 5-Fu in short), decitabine, gemcitabine, carmofur, capecitabine, doxifluridine, tegafur and azacitidine.

In one embodiment of the present invention, the platinum-based chemotherapeutic drug is selected from the group consisting of cisplatin, carboplatin, nedaplatin, oxaliplatin and lobaplatin.

In one embodiment of the present invention, the paclitaxel-based chemotherapeutic drug is selected from the group consisting of paclitaxel and docetaxel.

In one embodiment of the present invention, the camptothecin-based chemotherapeutic drug is selected from the group consisting of camptothecin, hydroxylcamptothecine, topotecan and irinotecan.

In one embodiment of the present invention, the chemotherapeutic drug includes a pyrimidine analogue-based chemotherapeutic drug and a platinum-based chemotherapeutic drug. The pyrimidine analogue-based chemotherapeutic drug is selected from the group consisting of 5-fluorouracil, capecitabine and tegafur; and the platinum-based chemotherapeutic drug is selected from the group consisting of oxaliplatin and cisplatin.

In one embodiment of the present invention, the immune checkpoint inhibitor is one or more selected from the group consisting of an anti-PD-1 antibody, an anti-PD-L1 antibody, an anti-CTLA-4 antibody, a bispecific antibody including the anti-PD-1 antibody, a bispecific antibody including the anti-PD-L1 antibody, and a bispecific antibody including the anti-CTLA-4 antibody. As an example, the anti-PD-1 antibody includes, but is not limited to, nivolumab, pembrolizumab, toripalimab, sintilimab, camrelizumab, tislelizumab, penpulimab, zimberelimab, serplulimab, pucotenlimab, dostarlimab, cemiplimab and prolgolimab. As an example, the anti-PD-L1 antibody includes, but is not limited to, durvalumab, atezolizumab, envafolimab, sugemalimab, adebrelimab and avelumab.

In one embodiment of the present invention, the immune checkpoint inhibitor is selected from the group consisting of nivolumab, pembrolizumab, toripalimab, sintilimab, camrelizumab, tislelizumab, penpulimab, zimberelimab, serplulimab, pucotenlimab, dostarlimab, cemiplimab, prolgolimab, durvalumab, atezolizumab, envafolimab, sugemalimab, adebrelimab and avelumab.

In one embodiment of the present invention, the immune checkpoint inhibitor is selected from the group consisting of sintilimab and tislelizumab.

In one embodiment of the present invention, the tumor disease is selected from the group consisting of Claudin 18.2 positive cancers. In one embodiment of the present invention, the tumor disease is selected from the group consisting of gastric cancer, esophageal cancer and pancreatic cancer. In one embodiment of the present invention, the tumor disease is selected from the group consisting of Claudin 18.2-positive gastric cancer, esophageal cancer and pancreatic cancer. In the present invention, with reference to Standardization for diagnosis and treatment of gastric cancer (2022 editi*on)*, the gastric cancer includes gastroesophageal junction cancer (the gastroesophageal junction may also be written as the esophagogastric junction or the boundary between the stomach and the esophagus), see the Notice of the General Office of the National Health Commission of China on the Issuance of the Guidelines for the Diagnosis and Treatment of Tumors and Hematological Diseases (2022 Edition) (National Health Commission Medical Letter [2022] No. 104).

In one embodiment of the present invention, in the combination, the Claudin 18.2 antibody, the chemotherapeutic drug and the immune checkpoint inhibitor are administered simultaneously or at different times.

In one embodiment of the present invention, the Claudin 18.2 antibody is administered in a therapeutically effective amount, which may be provided in one or more dosage strengths. In one embodiment of the present invention, the Claudin 18.2 antibody is administered at a frequency of once a week, once every two weeks, once every three weeks, once a month, once every two months or twice every three weeks. In one embodiment of the present invention, the Claudin 18.2 antibody is administered parenterally.

In one embodiment of the present invention, the Claudin 18.2 antibody is administered at a dose of 2 mg/kg to 30 mg/kg. In one embodiment of the present invention, the Claudin 18.2 antibody is administered at a dose of 3 mg/kg to 25 mg/kg. In one embodiment of the present invention, the Claudin 18.2 antibody is administered at a dose of 4 mg/kg to 20 mg/kg. In one embodiment of the present invention, the Claudin 18.2 antibody is administered at a dose of 5 mg/kg to 15 mg/kg. In one embodiment of the present invention, the Claudin 18.2 antibody is administered at a dose of 6 mg/kg to 10 mg/kg. In one embodiment of the present invention, the Claudin 18.2 antibody is administered at a dose of 6 mg/kg, 8 mg/kg or 10 mg/kg.

In one embodiment of the present invention, the Claudin 18.2 antibody is administered at a dose of 100 mg/m² to 1,000 mg/m². In one embodiment of the present invention, the Claudin 18.2 antibody is administered at a dose of 150 mg/m² to 800 mg/m². In one embodiment of the present invention, the Claudin 18.2 antibody is administered at a dose of 200 mg/m² to 600 mg/m².

In one embodiment of the present invention, the Claudin 18.2 antibody is administered at a fixed dose that does not change with the body weight (or body surface area) of each patient; and the fixed dose is 100 mg to 1,500 mg or 300 mg to 1,000 mg.

In one embodiment of the present invention, the chemotherapeutic drug is administered in a therapeutically effective amount.

In one embodiment of the present invention, the chemotherapeutic drug includes oxaliplatin and capecitabine. An exemplary dosage regimen is as follows: 130 mg/m² of oxaliplatin, intravenous administration on Day 1; 1,000 mg/m² of capecitabine, oral administration twice daily from Days 1 to 14; repeated every 21 days.

In one embodiment of the present invention, the chemotherapeutic drug includes oxaliplatin and tegafur. An exemplary dosage regimen is as follows: 130 mg/m² of oxaliplatin, intravenous administration on Day 1; 80 mg/m² of S-1, oral administration twice daily from Days 1 to 14; repeated every 21 days. S-1 includes tegafur, gimeracil, and oteracil, and is exemplarily the Tegafur, Gimeracil and Oteracil Potassium Capsule.

In one embodiment of the present invention, the chemotherapeutic drug includes oxaliplatin, 5-fluorouracil and the chemotherapeutic drug leucovorin calcium. An exemplary dosage regimen is as follows: 85 mg/m² of oxaliplatin, intravenous administration on Day 1; 400 mg/m² of leucovorin calcium, intravenous administration on Day 1; 400 mg/m² of 5-fluorouracil, intravenous administration on Day 1; 5-fluorouracil, 2,400 mg/(m²·d) to 3,600 mg/(m²·d), continuous intravenous administration for 48 hours; repeated every 14 days.

In one embodiment of the present invention, the chemotherapeutic drug is docetaxel. An exemplary dosage regimen is as follows: 75 mg/m² to 100 mg/m² of docetaxel, intravenous administration on Day 1; repeated every 21 days.

In one embodiment of the present invention, the chemotherapeutic drug is paclitaxel. An exemplary dosage regimen is as follows: 80 mg/m² of paclitaxel, intravenous administration on Days 1, 8, and 15; repeated every 28 days. Another exemplary dosage regimen is as follows: 135 mg/m² to 250 mg/m² of paclitaxel, intravenous administration on Day 1; repeated every 21 days.

In one embodiment of the present invention, the chemotherapeutic drug is irinotecan. An exemplary dosage regimen is as follows: 125 mg/m² of irinotecan, intravenous administration on Days 1 and 8; repeated every 21 days. Another exemplary dosage regimen is as follows: 150 mg/m² to 180 mg/m² of irinotecan, intravenous administration on Day 1; repeated every 14 days.

In one embodiment of the present invention, the immune checkpoint inhibitor is administered in a therapeutically effective amount, which may be provided in one or more dosage strengths. In one embodiment of the present invention, the immune checkpoint inhibitor is administered at a frequency of once a week, once every two weeks, once every three weeks, once a month, once every two months or twice every three weeks. In one embodiment of the present invention, the immune checkpoint inhibitor is administered parenterally.

In one embodiment of the present invention, the immune checkpoint inhibitor is nivolumab. An exemplary dosage regimen is as follows: 3 mg/kg or 240 mg of nivolumab, intravenous administration on Day 1; repeated every 14 days. Another exemplary dosage regimen is as follows: 360 mg of nivolumab, intravenous administration on Day 1; repeated every 21 days. Another exemplary dosage regimen: 480 mg of nivolumab, intravenous administration on Day 1; repeated every 28 days.

In one embodiment of the present invention, the immune checkpoint inhibitor is pembrolizumab. An exemplary dosage regimen is as follows: 200 mg of pembrolizumab, intravenous administration on Day 1; repeated every 21 days. Another exemplary dosage regimen is as follows: 400 mg of pembrolizumab, intravenous administration on Day 1; repeated every 42 days.

In one embodiment of the present invention, the immune checkpoint inhibitor is sintilimab. An exemplary dosage regimen is as follows: 200 mg of sintilimab, intravenous administration on Day 1; repeated every 21 days. Another exemplary dosage regimen is as follows: 3 mg/kg sintilimab, intravenous administration on Day 1; repeated every 21 days.

In one embodiment of the present invention, the immune checkpoint inhibitor is tislelizumab. An exemplary dosage regimen is as follows: 200 mg of tislelizumab, intravenous administration on Day 1; repeated every 21 days.

Those skilled in the art can easily determine the CDR sequence based on the sequence of the antibody heavy chain or light chain variable region. To define the CDR of the antibody variable region, it is necessary to choose an antibody numbering scheme and a definition scheme. The numbering scheme includes, but is not limited to: IMGT, Chothia, Kabat, or Martin (Enhanced Chothia); and the definition scheme includes, but is not limited to, Chothia, Kabat, IMGT, or Contact.

The term "combination" of medicaments includes a fixed-dose combination of medicaments (in a single dosage form) and a combination package of medicaments for combined administration, and also includes a combination therapy of medicaments. In the case of the combination package of medicaments, or the combination therapy of medicaments, each medicament in which may be administered simultaneously or separately within specific time intervals. In the present invention, the term "fixed-dose combination" refers to a single dosage form formulated to deliver a certain amount of two or more medicaments to a patient; and exemplary examples include a compound preparation and a composite preparation. In the present invention, the term "compound preparation" or "composite preparation" refers to a medicine composed of several pharmaceutical ingredients, in which the "compound preparation" refers to a preparation that is mixed with several different types of medicaments; and the "composite preparation" refers to a preparation that is composed of several medicaments of the same type, and it is allowed that a composite preparation contains other types of medicaments. In the present invention, the term "combination package of medicaments" refers to a package composed of two or more pharmaceutical preparations with independent indications, usages and dosages. In the present invention, the term "combination therapy" of medicaments refers to the administration of two or more medicaments with independent indications, usages and dosages to a patient for the treatment of related diseases. In the case of the "combination package of medicaments" or the "combination therapy" of medicaments, as an example, the Claudin 18.2 antibody may be administered first, optionally followed by the immune checkpoint inhibitor, and then the chemotherapeutic drug.

The term "pharmaceutically acceptable" refers to substances, materials, compositions and/or dosage forms that, within the scope of reliable medical judgment, are suitable for use in contact with the human and animal tissues without undue toxicity, irritation, allergic reactions or other problems or complications, and are commensurate with a reasonable benefit/risk ratio.

The term "therapeutically effective amount" refers to an amount that is effective to treat or inhibit a corresponding disease or disorder, thereby producing a desired therapeutic, ameliorative, inhibitory or prophylactic effect. For the administered dose of an active ingredient (including but not limited to Claudin 18.2 antibodies, chemotherapeutic drugs, and immune checkpoint inhibitors) involved in the present invention, unless otherwise specified, "mg/kg" means milligrams (or mg) of the active ingredient administered per kilogram of body weight, and "mg/m²" means milligrams of the active ingredient administered per square meter of body surface area. Those skilled in the art know the method for calculating the body surface area, for example, according to Stevenson's formula, DuBois's formula, Mosteller's formula, Haycock's formula, and the like.

The term "intravenous administration" refers to the injection of a fluid substance (including, but not limited to, blood, a medicinal solution, and a nutritional solution) into a vein. The intravenous administration can be classified into short-term intravenous administration and continuous intravenous administration. The short-term intravenous administration is usually performed by direct injection into a vein with a syringe; and the continuous intravenous administration is usually performed by intravenous infusion, including the case performed with the help of an infusion pump.

The term "containing" or "comprising (including)" can be open, semi-closed and closed. In other words, these terms also include "consisting essentially of" or "consisting of".

The term "Mass-volume percentage" or "% (w/v)" or "% w/v" is an expression of mass-volume concentration, indicating the number of grams of solute contained in every 100 ml of solution. For example, 20% (w/v) means that every 100 ml of solution contains 20 g of solute.

The terms "Claudin 18", "claudin 18" and "CLDN18" are interchangeable; and the terms "Claudin 18.2", "claudin 18.2", "CLDN18.2" and "CLDN18_2" are interchangeable.

The term "Optional" or "optionally" means that the subsequently described event or condition may but need not occur, and the description includes both instances where the stated event or condition occurs and instances where the stated event or condition does not occur.

Sequence alignment generally refers to aligning test sequences by taking one sequence as a reference sequence. In the present invention, the term "similarity" of sequences refers to the proportion of identical amino acids between a test sequence and a target sequence relative to the entire sequence (a relatively macroscopic description). In amino acid sequence alignment, similarity also includes, in addition to completely identical residues, whether two residues (except for identical residues) at corresponding positions possess similar characteristics, such as side-chain size, charge, hydrophilicity / hydrophobicity, and the like. The method for performing sequence alignment is common general knowledge in the art.

### Beneficial effects of the present invention:

(1) the combination of the Claudin 18.2 antibody and the chemotherapeutic drug has a synergistic effect in tumor treatment as compared to monotherapy with the Claudin 18.2 antibody or the chemotherapy alone;
(2) the combined use of the Claudin 18.2 antibody, the chemotherapeutic drug and the anti-PD-1 monoclonal antibody exhibits manageable safety and good anti-tumor activity.

As an example, the Claudin 18.2 antibody is a humanized monoclonal IgG1 antibody targeting the target Claudin 18.2, with a heavy chain as set forth in SEQ ID NO: 9, and a light chain as set forth in SEQ ID NO: 10.

### Sequence information

The information of some sequences involved in the present disclosure is as follows:
SEQ ID NO: 1 (heavy chain CDR1 of Claudin 18.2 antibody)
   GYTFTSYVIN
SEQ ID NO: 2 (heavy chain CDR2 of Claudin 18.2 antibody)
   EIHPRGGNTYYSEKFRG
SEQ ID NO: 3 (heavy chain CDR3 of Claudin 18.2 antibody)
   LRRGNAMDY
SEQ ID NO: 4 (light chain CDR1 of Claudin 18.2 antibody)
   KSSQSLLNSGNQRNYLT
SEQ ID NO: 5 (light chain CDR2 of Claudin 18.2 antibody)
   WASTRES
SEQ ID NO: 6 (light chain CDR3 of Claudin 18.2 antibody)
   QNSYNYPYT
SEQ ID NO: 7 (heavy chain variable region of Claudin 18.2 antibody) 
QVQLVQSGAEVKKPGASVKVSCKASGYTFTSYVINWVRQATGQGLEWIGEIH
PRGGNTYYSEKFRGRVTLTADTSISTAYMELSSLRSEDTAVYYCARLRRGNAM
DYWDQGTTVTVSS
SEQ ID NO: 8 (light chain variable region of Claudin 18.2 antibody) 
DIVMTQSPSSLAVSLGEMATINCKSSQSLLNSGNQRNYLTWYQQKPGQPPKLLI
YWASTRESGVPDRFSGSGSGTDFTLTISSLQAEDVAVYYCQNSYNYPYTFGQG
TKLERK
SEQ ID NO: 9 (heavy chain of Claudin 18.2 antibody) 
QVQLVQSGAEVKKPGASVKVSCKASGYTFTSYVINWVRQATGQGLEWIGEIH
PRGGNTYYSEKFRGRVTLTADTSISTAYMELSSLRSEDTAVYYCARLRRGNAM
DYWDQGTTVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTKV
DKKVEPKSCDKTHTCPPCPAPELLGGPSVFLFPPKPKDTLMISRTPEVTCVVVD
VSHEDPEVKFNWYVDGVEVHNAKTKPREEQYNSTYRVVSVLTVLHQDWLNG
KEYKCKVSNKALPAPIEKTISKAKGQPREPQVYTLPPSRDELTKNQVSLTCLVK
GFYPSDIAVEWESNGQPENNYKTTPPVLDSDGSFFLYSKLTVDKSRWQQGNVF
SCSVMHEALHNHYTQKSLSLSPGK
SEQ ID NO: 10 (light chain of Claudin 18.2 antibody)

DIVMTQSPSSLAVSLGEMATINCKSSQSLLNSGNQRNYLTWYQQKPGQPPKLLI
YWASTRESGVPDRFSGSGSGTDFTLTISSLQAEDVAVYYCQNSYNYPYTFGQG
TKLERKRTVAAPSVFIFPPSDEQLKSGTASVVCLLNNFYPREAKVQWKVDNAL
QSGNSQESVTEQDSKDSTYSLSSTLTLSKADYEKHKVYACEVTHQGLSSPVTK
SFNRGEC

### SPECIFIC MODELS FOR CARRYING OUT THE PRESENT DISCLOSURE

The present invention is further described below in conjunction with specific examples. It should be understood that these examples are intended only to illustrate the present invention, rather than limiting the scope of the present invention. In the examples below, experimental methods without clear indication of specific conditions are generally carried out following the conventional conditions or the conditions recommended by the manufacturers.

Unless otherwise specified, the abbreviations used in the present invention have the following meanings:
M5 monoclonal antibody: a humanized monoclonal IgG1 antibody targeting the target Claudian 18.2, with a heavy chain as set forth in SEQ ID NO: 9, and a light chain as set forth in SEQ ID NO: 10.
CAPOX: a chemotherapy regimen including oxaliplatin and capecitabine, also called XELOX regimen;
DS-3: an anti-Claudin 18.2 antibody disclosed in CN118307678A, which can be used to prepare *in vitro* detection reagents;
BIW: twice weekly;
Q4D: once every four (4) days; and
Q3W: once every three (3) weeks.

Unless otherwise specified, in the mouse efficacy studies involved in the following Examples 1, 2, and 3:
the tumor volume (V) is calculated according to the formula: V = 1/2 × a × b², where "a" and "b" represent the length (long diameter) and width (short diameter) of a tumor, respectively;
   $T / C \left(%\right) = \left(T-{T}_{0}\right) / \left(C-{C}_{0}\right) \times 100 % ,$ where T and C each indicate the tumor volume on a specific day in the experiment, and T₀ and C₀ each indicate the tumor volume at the beginning of the experiment, and T and T₀ correspond to the treatment group, and C and C₀ correspond to the negative control group; and
tumor growth inhibition (TGI) (%) = 100% - T/C (%).

### Example 1: Efficacy against Subcutaneous Xenograft Tumors of Human Gastric Cancer KATO III/18.2 in Mice

Human gastric cancer KATO III cells, purchased from the Cell Bank of the Chinese Academy of Sciences, were transfected with the human Claudin 18.2 gene to achieve high expression of Claudin 18.2 proteins, then designated as KATO III/18.2. NOD-Scid mice (7 weeks old, female) were purchased from Shanghai Lingchang Biotechnology Co., Ltd. Each mouse was subcutaneously inoculated with 1 × 10⁷ KATO III/18.2 cells. When tumor volume reached 60 mm³ to 150 mm³, the mice were grouped according to tumor volume, and then the medicaments were administered intraperitoneally (IP) at an injection volume of 0.1 mL per 10 g of body weight.

**Table 1**

| | |
|---|---|
| Dosage regimen for negative control group | Human IgG1 (10 mg/kg, IP, BIW, 6 times in total) |
| Dosage regimen for oxaliplatin monotherapy group | Human IgG1 (10 mg/kg, IP, BIW, 6 times in total) |
| | Oxaliplatin (8 mg/kg, IP, Q4D, 3 times in total) |
| Dosage regimen for the combination therapy group | M5 monoclonal antibody (10 mg/kg, IP, BIW, 6 times in total) |
| of M5 monoclonal antibody and oxaliplatin | Oxaliplatin (8 mg/kg, IP, Q4D, 3 times in total) |

The tumor growth inhibition of the oxaliplatin monotherapy against KATO III/18.2 was 31%; and the tumor growth inhibition of the combination therapy group of M5 monoclonal antibody and oxaliplatin was 82%. These measurement results were obtained on Day 23 (with the first dose administered on Day 0). The combination of the M5 monoclonal antibody and oxaliplatin significantly improved the chemotherapeutic efficacy of oxaliplatin.

In the oxaliplatin monotherapy group and the combination therapy group of oxaliplatin and M5 monoclonal antibody, the maximum weight losses were 5.5% (on Day 10) and 7.0% (on Day 10) in mice, respectively.

### Example 2: Efficacy against Subcutaneous Xenograft Tumors in Human Pancreatic Cancer Miapaca2/18.2 Mice

Miapaca2 cells, purchased from American Type Culture Collection (ATCC), were transfected with the human Claudin 18.2 gene to achieve high expression of human Claudin 18.2 proteins, then designated as Miapaca2/18.2. NOD-Scid mice (7 weeks old, female) were purchased from Shanghai Lingchang Biotechnology Co., Ltd. Each mouse was subcutaneously inoculated with 1 × 10⁷ Miapaca2/18.2 cells. When tumor volume reached 60 mm³ to 150 mm³, the mice were grouped according to tumor volume and then the medicaments were administered intraperitoneally (IP) at an injection volume of 0.1 mL per 10 g of body weight.

**Table 2**

| | |
|---|---|
| Dosage regimen for negative control group | Human IgG1 (10 mg/kg, IP, BIW, 4 times in total) |
| Dosage regimen for the antibody monotherapy group of M5 monoclonal | M5 monoclonal antibody (10 mg/kg, IP, BIW, 4 times in total) |
| Dosage regimen for the monotherapy group of gemcitabine | Human IgG1 (10 mg/kg, IP, BIW, 4 times in total) |
| | Gemcitabine (75 mg/kg, IP, Q4D, 2 times in total) |
| Dosage regimen for the combination therapy group | M5 monoclonal antibody (10 mg/kg, IP, BIW, 4 times in total) |
| of M5 monoclonal antibody and gemcitabine | Gemcitabine (75 mg/kg, IP, Q4D, 2 times in total) |

The M5 monoclonal antibody monotherapy showed an inhibitory effect on the growth of subcutaneously xenografted human pancreatic cancer MiaPaCa2/18.2 tumors in mice, with a tumor growth inhibition of 26%; the gemcitabine monotherapy showed a tumor growth inhibition of 53% against Miapaca2/18.2; and the combination therapy of M5 monoclonal antibody and gemcitabine increased the tumor growth inhibition to 64%. These measurement results were obtained on Day 14 (with the first dose administered on Day 0). The results demonstrated that the combination of M5 monoclonal antibody and the gemcitabine exerted the synergistic effect.

In the gemcitabine monotherapy group, the maximum weight loss in mice was 9.2% (on Day 7), with one out of ten mice died; and in the combination group of gemcitabine and M5 monoclonal antibody, the maximum weight loss in mice was 12.2% (on Day 7), with no mouse died.

### Example 3: Anti-tumor Efficacy in C57BL/6 Mice Bearing Subcutaneous MC38-hClaudin18.2 Tumors

C57BL/6 mice (6 to 8 weeks old, female) were purchased from Shanghai Model Organisms Center, Inc. Each mouse was subcutaneously inoculated with 1 × 10⁶ MC38-hClaudin18.2 cells in the right flank. When the average tumor volume reached approximately 89 mm³, the mice were randomly grouped according to tumor volume, with 10 mice per group. Administration was initiated on the day of grouping, which was designated as Day 0.

**Table 3**

| Group | Administration | Dose (mg/kg) | Frequency |
|---|---|---|---|
| Negative control | PBS | / | BIW, 6 times |
| M5 monoclonal antibody | M5 monoclonal antibody | 10.0 | BIW, 6 times |
| Murine anti-PD-1 antibody | Murine anti-PD-1 antibody | 3.0 | BIW, 6 times |
| Combination | M5 monoclonal | 10.0 + 3.0 | BIW, 6 times |
| therapy group | antibody and Murine anti-PD-1 antibody | | |

On Day 21 after the initiation of administration in the MC38-hClaudin18.2 xenograft tumor (colon cancer) model, the tumor growth inhibition of the M5 monoclonal antibody group, the murine anti-PD-1 group and the combination therapy group were 86.91%, 80.89% and 103.63%, respectively. The tumor volume of the combination therapy group was 9.13 ± 5.07 mm³, and the tumor volume in the negative control group was 2,311.77 ± 372.63 mm³. Complete tumor regression was observed in 7 out of 10 mice in the combination therapy group, whereas only 1 out of 10 mice in the M5 monoclonal antibody group and 2 out of 10 mice in the murine anti-PD-1 group achieved complete tumor regression.

In terms of safety, the combination therapy does not lead to a significant increase in toxicity (without difference in animal weight change and mortality).

### Example 4: Clinical Trial Study (Combination Therapy of M5 Monoclonal Antibody and CAPOX)

M5 monoclonal antibody injection at doses of 3 mg/kg (n = 4), 6 mg/kg (n = 3), 10 mg/kg (n = 4), and 15 mg/kg (n = 7) was combined with CAPOX for treatment to investigate Cₘₐₓ and AUC₍₀₋ₜ₎ in the serum of patients. The mean Cₘₐₓ of the 6 mg/kg, 10 mg/kg and 15 mg/kg groups were 1.9-fold, 4.1-fold, and 6.0-fold that of the 3 mg/kg group, respectively; and the mean AUC₍₀₋ₜ₎ of the 6 mg/kg, 10 mg/kg and 15 mg/kg groups were 2.17-fold, 2.53-fold and 5.05-fold that of the 3 mg/kg group, respectively.

In the case of the combination therapy of M5 monoclonal antibody and CAPOX, the M5 monoclonal antibody showed linear pharmacokinetics (PK) in the dose range of 3 mg/kg to 15 mg/kg. The PK of the M5 monoclonal antibody in combination with CAPOX was substantially consistent with that of M5 monoclonal antibody monotherapy, indicating that the CAPOX therapy had no effect on the PK of the M5 monoclonal antibody.

Dosage of M5 monoclonal antibody/placebo: The M5 monoclonal antibody/placebo was administered once every 3 weeks, with a 21-day treatment cycle, the M5 monoclonal antibody/placebo was infused on the first day of each treatment cycle.

CAPOX chemotherapy regimen: 130 mg/m² of oxaliplatin, intravenous administration on Day 1; 1,000 mg/m² of capecitabine, oral administration twice daily from Days 1 to 14; repeated every 21 days.

### Example 5: Treatment of Gastric Cancer or Gastroesophageal Junction Adenocarcinoma with the Combination Therapy of M5 Monoclonal Antibody and CAPOX

Patients with advanced recurrent or metastatic gastric cancer or gastroesophageal junction adenocarcinoma (stage IV according to TNM staging, none of whom had received any prior systemic treatment for advanced disease), who had been enrolled in the study, showed moderate to high expression of Claudin 18.2.

Based on the response evaluation criteria in solid tumors (RECIST1.1), the confirmed tumor objective response rate (ORR) and disease control rate (DCR) were presented in the following table. The 6 mg/kg group refers to that the M5 monoclonal antibody was intravenously administered at a dose of 6 mg/kg once every three weeks, in combination with the CAPOX chemotherapy regimen. The 10 mg/kg group in Table 4 refers to that the M5 monoclonal antibody was intravenously administered at a dose of 10 mg/kg once every three weeks, in combination with the CAPOX chemotherapy regimen.

**Table 4**

| EFR set | 6 mg/kg (N = 30) | 10 mg/kg (N = 20) |
|---|---|---|
| Complete response (CR) | 1 (3.3%) | 0 (0.0) |
| Partial response (PR) | 17 (56.7%) | 12 (60%) |
| Stable disease (SD) | 10 (33.3%) | 6 (30%) |
| Progressive disease (PD) | 2 (6.7%) | 2 (10%) |
| ORR (%) | 60% | 60% |
| DCR (%) | 93.3% | 90% |

The inventor unexpectedly found that the efficacy data showed that the therapeutic effect in the 6 mg/kg group was comparable to that in the 10 mg/kg group.

The safety data were shown in the table below. As indicated in the safety data in Table 5, the side effects in the patients in the 6 mg/kg dose group was lower than that in the 10 mg/kg group.

**Table 5**

| | | | | | | |
|---|---|---|---|---|---|---|
| Common TEAEs | M5 monoclonal | M5 monoclonal | Zolbetuximab + | | | |

| N (%) | antibody + CAPOX 6 mg/kg (n = 48) | | antibody + CAPOX 10 mg/kg (n = 36) | | CAPOX¹ (n = 254) | |
|---|---|---|---|---|---|---|
| | Any Grade | ≥3 Grade | Any Grade | ≥3 Grade | Any Grade | ≥3 Grade |
| Nausea | 28 (58.3) | 0 (0.0) | 24 (66.7) | 1 (2.8) | 68.5% | 8.7% |
| Vomiting | 25 (52.1) | 1 (2.1) | 22 (61.1) | 3 (8.3) | 66.1% | 12.2% |
| Decreased appetite | 21 (43.8) | 0 (0.0) | 10 (27.8) | 0 (0.0) | 41.3% | 6.7% |
| Hypoalbuminemia | 26 (54.2) | 4 (8.3) | 25 (69.4) | 4 (11.1) | 22.4% | 3.1% |
| Neutropenia | 18 (37.5) | 7 (14.6) | 15 (41.7) | 3 (8.3) | 19.7% | 7.1% |
| Edema | 1 (2.1) | 0 (0.0) | 2 (5.6) | 0 (0.0) | <15% | NA |
| Loss of weight | 14 (29.2) | 1 (2.1) | 11 (30.6) | 0 (0.0) | 19.7% | 0.5% |
| Note 1: Nature Medicine volume 29, pages 2,133-2,141 (2023). | | | | | | |

In another clinical study, among the enrolled 31patients with gastric cancer or gastroesophageal junction cancer and 9 patients with other solid tumors, 21 patients with gastric cancer or gastroesophageal junction cancer who received the M5 monoclonal antibody monotherapy at a dose of ≥10 mg/kg underwent at least one tumor efficacy evaluation in accordance with the RECIST 1.1 standards. Among the patients that underwent the evaluation, 6 patients were evaluated as SD, and 2 patients were evaluated as PR at first tumor evaluation.

It showed that the combination therapy of M5 monoclonal antibody and CAPOX exerted a synergistic effect in the treatment of gastric cancer or gastroesophageal junction adenocarcinoma as compared to the M5 monoclonal antibody monotherapy and the CAPOX therapy alone (a chemotherapy regimen in the prior art).

### Example 6: Second-line Treatment of gastric cancer or gastroesophageal junction adenocarcinoma with the Combination Therapy of M5 Monoclonal Antibody and Chemotherapy

Preliminary efficacy and safety data of M5 monoclonal antibody at a dose of 6 mg/kg combined with chemotherapy for second-line treatment of patients with gastric cancer or gastroesophageal junction adenocarcinoma (G/GEJ adenocarcinoma).

### Study protocol

This study was designed to evaluate the safety and efficacy of the M5 monoclonal antibody combined with paclitaxel or other guideline-recommended chemotherapeutic drugs as the second-line treatment for advanced G/GEJ adenocarcinoma. Claudin 18.2 expression was assessed by immunohistochemistry (IHC) analysis using a DS-3 detection reagent in a central laboratory. Patients (Pts) with positive expression of Claudin 18.2 (defined as membrane staining intensity ≥1+ in any tumor cell) were enrolled.

Efficacy was assessed by RECIST 1.1 every 2 cycles (6 weeks). Adverse events (AEs) were graded using CTCAE v5.0.

### Results

47 patients with G/GEJ adenocarcinoma received the combination therapy of M5 monoclonal antibody (at a dose of 6 mg/kg) and chemotherapy as second-line treatment. At baseline, the median age was 58.0 years old (in a range of 28 to 76 years old), and 57.4% (27/47) of patients had previously received immune checkpoint inhibitor therapy.

37 patients with moderate to high expression of Claudin 18.2, who had at least one post-baseline tumor assessment, were enrolled in the EFR group. 13 patients were evaluated as partial response (PR) (with a confirmed ORR of 35.1%), and 26 patients were evaluated as stable disease (SD) (with a disease control rate (DCR) of 70.3%). Patients with high expression of Claudin 18.2 showed higher tumor response rates (11 out of 29 patients, with a confirmed ORR of 37.9%), with the median duration of response (mDOR) of 4.07 months (95% CI: 2.63, 8.25) and the median progression-free survival (mPFS) of 5.29 months (95% CI: 2.66, 7.06). The overall survival (OS) of those with high expression of Claudin 18.2 showed a positive trend.

Such therapies usually showed good tolerance. Hypoalbuminemia, gastrointestinal reactions and hematological toxicities were the most frequently reported adverse events (AEs), all of which were related to the mechanism of action of Claudin 18.2 antibodies or chemotherapies.

### Conclusion

The M5 monoclonal antibody showed good clinical efficacy in G/GEJ adenocarcinoma patients with high expression of Claudin 18.2. The safety of the combination therapy of M5 monoclonal antibody and chemotherapy as second-line treatment was manageable. These results provided a theoretical basis for further exploration of the second-line treatment for patients with G/GEJ adenocarcinoma.

### Example 7

In the ongoing phase-Ib/II study, the PK of M5 monoclonal antibody (at a dose of 6 mg/kg or 10 mg/kg) in combination with CAPOX and anti-PD-1 antibody was consistent with that of M5 monoclonal antibody monotherapy, indicating that the combination therapy (M5 monoclonal antibody + CAPOX chemotherapy + anti-PD1 antibody) had no effect on the PK of the M5 monoclonal antibody.

### Example 8: Efficacy of the Therapy Combining M5 Monoclonal Antibody (at a dose of 6 mg/kg or 10 mg/kg) with CAPOX and anti-PD-1 Antibody

In the ongoing phase-Ib/II study, the M5 monoclonal antibody (at a dose of 6 mg/kg or 10 mg/kg) was combined with CAPOX and the PD1 antibody to treat patients with gastric cancer or gastroesophageal junction adenocarcinoma (G/GEJ adenocarcinoma).

### Study design

This study enrolled G/GEJ adenocarcinoma patients with positive expression of Claudin 18.2. In the dose escalation phase, a 3 + 3 design was used to determine the maximum tolerated dose (MTD). In the expansion phase, the doses were selected based on the safety, tolerability pharmacokinetics and antitumor activity in the escalation phase. Efficacy was assessed according to RECIST 1.1 every 2 cycles (6 weeks). Adverse events (AEs) were graded using CTCAE v5.0.

During the dose escalation process, patients received intravenous (IV) administration of M5 monoclonal antibodies at doses of 6 mg/kg (n = 3) and 10 mg/kg (n = 6) every 3 weeks (Q3W) in combination with CAPOX and sintilimab. In the expansion phase, all patients received IV administration of M5 monoclonal antibodies at a dose of 6 mg/kg.

### Results

As of July 20, 2023, 9 patients had been enrolled in the escalation phase. No DLT (dose-limiting toxicity) was observed, and thus, the MTD (maximum tolerated dose) was not determined. Treatment-related adverse events (TRAEs) occurred in 9 patients (100%), with the most common TRAEs being nausea (77.8%), hypoalbuminemia (66.7%) and hyponatremia (55.6%). Although most TRAEs were of Grade 1 or 2, one patient (11.1%) had a Grade-3 TRAE (neutropenia [10 mg/kg]).

In the expansion phase, 26 patients with moderate to high expression of Claudin 18.2 (with ≥40% of the tumor cells exhibiting membrane staining intensity ≥2+) were enrolled in the safety group. The TRAEs occurred in 22 patients (84.6%), with the most common TRAEs being nausea (57.7%), vomiting (53.8%), hypoalbuminemia (53.8%), hyponatremia (38.5%), fatigue (26.9%) and neutropenia (23.1%). Although most TRAEs were of Grade 1 or 2, three patients (11.5%) had TRAEs of ≥ Grade 3, including hypocalcemia, hypokalemia, neutropenia, fatigue and myelosuppression. Among the 15 evaluable patients who had at least one post-baseline tumor assessment, 12 patients (80.0%) achieved PR, with an unconfirmed objective response rate (ORR) of 80.0%; another 3 patients (20.0%) achieved SD; and the disease control rate (DCR) was 100.0%.

The PK of M5 monoclonal antibody at doses of 6 mg/kg and 10 mg/kg in combination with CAPOX and the anti-PD1 antibody was consistent with that of M5 monoclonal antibody monotherapy.

**Table 6**

| Common TEAEs N (%) | M5 monoclonal antibody + CAPOX 6 mg/kg (n = 48) | | M5 monoclonal antibody + CAPOX + PD-1 inhibitor 6 mg/kg (n = 26) | |
|---|---|---|---|---|
| | Any Grade | ≥3 Grade | Any Grade | ≥3 Grade |
| Nausea | 28 (58.3) | 0 (0.0) | 15 (57.7) | 0 (0.0) |
| Vomiting | 25 (52.1) | 1 (2.1) | 15 (57.7) | 0 (0.0) |
| Decreased appetite | 21 (43.8) | 0 (0.0) | 2 (7.7) | 0 (0.0) |
| Hypoalbuminemia | 26 (54.2) | 4 (8.3) | 14 (53.8) | 0 (0.0) |
| Neutropenia | 18 (37.5) | 7 (14.6) | 10 (38.5) | 2 (7.7) |
| Edema | 1 (2.1) | 0 (0.0) | 2 (7.7) | 0 (0.0) |
| Loss of weight | 14 (29.2) | 1 (2.1) | 4 (15.4) | 0 (0.0) |

The inventor unexpectedly found that the efficacy and safety data showed this triple-combination therapy containing the anti-PD-1 antibody achieved better efficacy in patients as well as a safety comparable to that of the dual-combination therapy containing the M5 antibody at a dose of 6 mg/kg and the chemotherapy.

### Conclusion

The combined use of the Claudin 18.2 antibody, CAPOX and sintilimab showed manageable safety and good anti-tumor activity. The dose of 6 mg/kg was selected as the recommended dose for the subsequent study.

### Example 9: Efficacy of the Therapy Combining M5 Monoclonal Antibody (at a dose of 6 mg/kg) with CAPOX and anti-PD-1 antibody

Previous study results showed that the M5 monoclonal antibody was well tolerated and safe at a dose up to 10 mg/kg and exhibited promising anti-tumor effects. The dose of 6 mg/kg was selected for use in further study. Efficacy and safety data of M5 monoclonal antibody (at a dose of 6 mg/kg) in combination with CAPOX and anti-PD-1 antibody as the first-line treatment in patients with G/GEJ adenocarcinoma were evaluated.

### Study protocol

This study was designed to evaluate the safety and efficacy of the M5 monoclonal antibody in combination with CAPOX and sintilimab or tislelizumab as initial therapy for advanced G/GEJ adenocarcinoma, regardless of the expression levels of Claudin 18.2 or PD-L1. The expressions of Claudin 18.2 and PD-L1 were assessed by immunohistochemistry (IHC) analysis using a DS-3 detection reagent and an E1L3N detection reagent in the central laboratory. All patients (pts) were intravenously administered with the M5 monoclonal antibody at a dose of 6 mg/kg. Efficacy was assessed by RECIST 1.1 every 2 cycles (6 weeks). Adverse events (AEs) were graded using CTCAE v5.0.

### Results

A total of 49 evaluable patients with the Claudin 18.2 moderate/high (M/H) expression (with≥40% of tumor cells exhibiting membrane staining intensity ≥2+) were enrolled in the EFR group. Regardless of the PD-L1 CPS status, a total of 40 patients (81.6%) achieved partial or complete response, with a confirmed objective response rate (ORR) of 73.5% (95% CI: 58.9, 85.1). The median duration of response (mDOR) was 11.14 months (6.93, NE). In addition, 3 patients (26.5%) were evaluated as stable disease (SD), and the disease control rate (DCR) was 100.0%. It was also observed that patients with a CPS score <5 benefited from the therapy, with a confirmed objective response rate (ORR) of 74.1% (23 out of 31 patients). Patients with Claudin 18.2 H/M expression showed favorable trends in terms of both progression-free survival (PFS) and overall survival (OS), with a median progression-free survival (mPFS) of more than 10 months, and a 12-month OS rate of 77.1% (95% CI: 61.2, 87.2).

The safety of the triple-combination therapy was substantially consistent with that of first-line treatment using the combination therapy of M5 monoclonal antibody and CAPOX in patients with G/GEJ adenocarcinoma, with the main manifestation of manageable off-target effects including hypoalbuminemia, nausea, vomiting, etc., most of which were rated as Grade 1 or 2.

### Conclusion

The combination of M5 monoclonal antibody with CAPOX and the anti-PD-1 antibody showed safety and good tolerance as an initial therapy in patients with G/GEJ adenocarcinoma. Regardless of the PD-L1 expression level, this therapy exhibited promising anti-tumor effects.

All documents mentioned herein are incorporated by reference into the present application, as if each document is cited separately as a reference. In addition, it should be understood that various modifications or transformations can be made to the present invention by those skilled in the art after reading the above disclosure of the present invention, and these equivalent forms shall also fall within the scope defined by the appended claims.

## Claims

1. Use of a combination of a Claudin 18.2 antibody and a chemotherapeutic drug in the preparation of a medicament for the treatment of a tumor disease,
the Claudin 18.2 antibody has a heavy chain CDR1 as set forth in SEQ ID NO: 1, a heavy chain CDR2 as set forth in SEQ ID NO: 2, a heavy chain CDR3 as set forth in SEQ ID NO: 3, a light chain CDR1 as set forth in SEQ ID NO: 4, a light chain CDR2 as set forth in SEQ ID NO: 5, and a light chain CDR3 as set forth in SEQ ID NO: 6;
the chemotherapeutic drug is one or more selected from the group consisting of a pyrimidine analogue-based chemotherapeutic drug, a platinum-based chemotherapeutic drug, a paclitaxel-based chemotherapeutic drug, and a camptothecin-based chemotherapeutic drug; and
the combination optionally further comprises an immune checkpoint inhibitor.

2. A method for the treatment of a tumor disease in a patient, comprising administering a combination of a Claudin 18.2 antibody and a chemotherapeutic drug to the patient, **characterized in that**
the Claudin 18.2 antibody has a heavy chain CDR1 as set forth in SEQ ID NO: 1, a heavy chain CDR2 as set forth in SEQ ID NO: 2, a heavy chain CDR3 as set forth in SEQ ID NO: 3, a light chain CDR1 as set forth in SEQ ID NO: 4, a light chain CDR2 as set forth in SEQ ID NO: 5, and a light chain CDR3 as set forth in SEQ ID NO: 6;
the chemotherapeutic drug is one or more selected from the group consisting of a pyrimidine analogue-based chemotherapeutic drug, a platinum-based chemotherapeutic drug, a paclitaxel-based chemotherapeutic drug, and a camptothecin-based chemotherapeutic drug; and
the combination optionally further comprises an immune checkpoint inhibitor.

3. A pharmaceutical combination for use in the treatment of a tumor disease, comprising a Claudin 18.2 antibody and a chemotherapeutic drug, **characterized in that**
the Claudin 18.2 antibody has a heavy chain CDR1 as set forth in SEQ ID NO: 1, a heavy chain CDR2 as set forth in SEQ ID NO: 2, a heavy chain CDR3 as set forth in SEQ ID NO: 3, a light chain CDR1 as set forth in SEQ ID NO: 4, a light chain CDR2 as set forth in SEQ ID NO: 5, and a light chain CDR3 as set forth in SEQ ID NO: 6;
the chemotherapeutic drug is one or more selected from the group consisting of a pyrimidine analogue-based chemotherapeutic drug, a platinum-based chemotherapeutic drug, a paclitaxel-based chemotherapeutic drug, and a camptothecin-based chemotherapeutic drug; and
the pharmaceutical combination optionally further comprises an immune checkpoint inhibitor.

4. The use according to claim 1, or the method according to claim 2, or the pharmaceutical combination according to claim 3, **characterized in that** the Claudin 18.2 antibody has a heavy chain variable region as set forth in SEQ ID NO: 7, and a light chain variable region as set forth in SEQ ID NO: 8.

5. The use according to claim 1, or the method according to claim 2, or the pharmaceutical combination according to claim 3, **characterized in that** the Claudin 18.2 antibody has a heavy chain as set forth in SEQ ID NO: 9, and a light chain as set forth in SEQ ID NO: 10.

6. The use according to claim 1, or the method according to claim 2, or the pharmaceutical combination according to claim 3, **characterized in that**
the pyrimidine analogue-based chemotherapeutic drug is selected from the group consisting of cytarabine, 5-fluorouracil, decitabine, gemcitabine, carmofur, capecitabine, doxifluridine, tegafur and azacitidine;
the platinum-based chemotherapeutic drug is selected from the group consisting of cisplatin, carboplatin, nedaplatin, oxaliplatin and lobaplatin;
the paclitaxel-based chemotherapeutic drug is selected from the group consisting of paclitaxel and docetaxel; and
the camptothecin-based chemotherapeutic drug is selected from the group consisting of camptothecin, hydroxylcamptothecine, topotecan and irinotecan.

7. The use according to claim 1, or the method according to claim 2, or the pharmaceutical combination according to claim 3, **characterized in that** the immune checkpoint inhibitor is one or more selected from the group consisting of an anti-PD-1 antibody, an anti-PD-L1 antibody, an anti-CTLA-4 antibody, a bispecific antibody comprising the anti-PD-1 antibody, a bispecific antibody comprising the anti-PD-L1 antibody, and a bispecific antibody comprising the anti-CTLA-4 antibody.

8. The use according to claim 1, or the method according to claim 2, or the pharmaceutical combination according to claim 3, **characterized in that** the immune checkpoint inhibitor is selected from the group consisting of nivolumab, pembrolizumab, toripalimab, sintilimab, camrelizumab, tislelizumab, penpulimab, zimberelimab, serplulimab, pucotenlimab, dostarlimab, cemiplimab, prolgolimab, durvalumab, atezolizumab, envafolimab, sugemalimab, adebrelimab and avelumab.

9. The use according to claim 1, or the method according to claim 2, or the pharmaceutical combination according to claim 3, **characterized in that** the tumor disease is selected from the group consisting of Claudin 18.2-positive cancers.

10. The use according to claim 1, or the method according to claim 2, or the pharmaceutical combination according to claim 3, **characterized in that** the tumor disease is selected from the group consisting of gastric cancer, esophageal cancer and pancreatic cancer.

11. The use according to claim 1, or the method according to claim 2, or the pharmaceutical combination according to claim 3, **characterized in that** the tumor disease is selected from the group consisting of Claudin 18.2-positive gastric cancer, esophageal cancer and pancreatic cancer.

12. The use according to claim 1, or the method according to claim 2, or the pharmaceutical combination according to claim 3, **characterized in that** the Claudin 18.2 antibody is intravenously administered at a dose of 6 mg/kg once every three weeks.
